(19) Europäisches Patentamt
European Patent Office
Office européen des brevets

(11)  **EP 3 141 181 B1**

(12)  **EUROPEAN PATENT SPECIFICATION**

(45) Date of publication and mention
of the grant of the patent:
**20.06.2018   Bulletin 2018/25**

(51) Int Cl.:
*A61B 5/00* *(2006.01)*          *A61B 17/00* *(2006.01)*
*A61B 18/00* *(2006.01)*          *A61B 18/14* *(2006.01)*

(21) Application number: **15184821.5**

(22) Date of filing: **11.09.2015**

(54) **ABLATION CATHETER APPARATUS WITH A BASKET COMPRISING ELECTRODES, AN OPTICAL EMITTING ELEMENT AND AN OPTICAL RECEIVING ELEMENT**

KATHETERVORRICHTUNG ZUR ABLATION MIT EINEM KORB UMFASSEND ELEKTRODEN, EIN OPTISCHES STRAHLELEMENT UND EIN OPTISCHES EMPFANGELEMENT

APPAREIL À CATHÉTER POUR L'ABLATION AVEC UN PANIER COMPRENANT DES ÉLECTRODES, UN ÉMETTEUR OPTIQUE ET UN RECEVEUR OPTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**15.03.2017   Bulletin 2017/11**

(73) Proprietor: **Lim, Bernard Boon Chye**
**Springfield, IL 62712 (US)**

(72) Inventor: **Lim, Bernard Boon Chye**
**Springfield, IL 62712 (US)**

(74) Representative: **FRKelly**
**27 Clyde Road**
**Dublin D04 F838 (IE)**

(56) References cited:
**US-A- 5 298 026          US-A1- 2008 125 634**
**US-A1- 2010 063 492      US-A1- 2011 028 837**

## Description

## Field of the Invention

The invention relates to an ablation catheter apparatus. A tissue composition monitoring apparatus, a tissue composition monitoring method and an ablation lesion determination automated algorithm are also described, the algorithm incorporating Arrhenius model thermal denaturation kinetics for determining the characteristics of tissue, for example the transmurality of the ablation lesion.

## Background of the Invention

Methods incorporating diffuse reflectance spectroscopy (DRS), into standard ablation catheters suffer from instability of collection of optical spectra due to motion of the catheter and inconsistent contact pressure. Furthermore, DRS deployed with a small offset between illuminating and collecting fibers have only small penetration depths. Standard ablation catheters cannot achieve a sufficient spatial offset between illuminating and receiving optic fibers due to the limited length of the distal tip of the ablation catheter that is in contact with the heart during ablation and therefore cannot exploit spatial offset to achieve greater penetration depths.

In ablation of cardiac tissue, the first hit is the most important. Therefore applying energy in the correct dosage and for the correct duration is critical to achieving transmurality. Insufficient energy and duration will lead to ineffective lesions which result in local tissue swelling and edema which will prevent further effective completion of the lesion at the same location. Checking for lesion characteristics after the first ablation is therefore ineffective. On the other hand, applying too much energy for too long can lead to collateral damage like coagulum formation and damage to surrounding structures. What is therefore needed is an apparatus to allow monitoring of the ablation lesion as it is being formed and to adjust power and duration settings according to predetermined optimum levels for achieving transmurality and for preventing collateral damage.

Description of related art

Systems which measure ablation lesions are known in the art. To quantify optical sensing data in a beating heart is challenging. There is substantial movement of the catheter. The translation of the catheter can be by as much as 1 cm within the timeframe of a single heartbeat. This movement can lead to significant error in spectrophotometer readings and even ultrasound images. The document US 2010/063492 A1 discloses an ablation catheter with a basket comprising energy emitting elements and sensors including optical sensors.

Harks et al., (WO 2012/049621 A1), hereinafter "Harks," incorporates optic fiber within a standard ablation catheter that is subject to cardiac motion which can lead to significant errors in spectrophotometer readings and is subject to different contact pressures which can lead to significant errors in spectrophotometer readings. Furthermore, in moving catheters around the cardiac atria there is a wide variation in the amount of pressure applied to the tissue. Kuck et.al; A novel radiofrequency ablation catheter using contact force sensing: Toccata study.

Heart Rhythm 2012 Jan;9(1): 18-23 hereinafter "Kuck," shows that there is large inter-individual and intra-individual variability in contact pressure of the ablation catheter within the left atrium. Even within a single individual, there is a wide variability in contact pressure as the catheter is moved from point to point in the heart. The contact pressure can differ by as much as up to 0.39 N (40 g) as the ablation catheter is moved around the pulmonary veins. In addition, depending in the individual clinician performing the ablation, a large variation in contact force up to 0.59 N (60 g) is possible.

Irrespective of the source, variability in pressure against tissues can influence measurement of optical sensing data. Variations in pressure on tissue can lead to variations in scattering and absorbance coefficients by as much as up to 25 % due to extrusion of water from tissue.

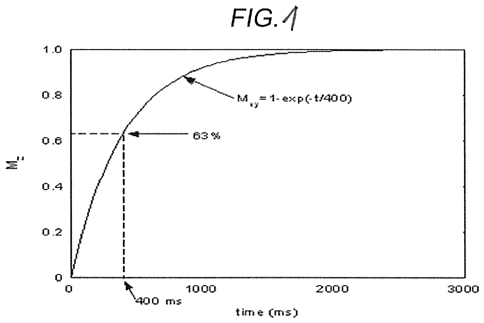

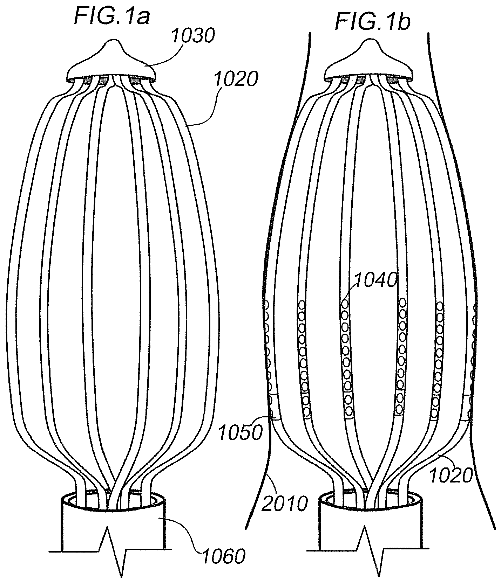

Accordingly, a preferred apparatus provides a stable platform having a basket shape with distal splines hugging the inside of the pulmonary vein and with the conical part of the basket straddling the os of the pulmonary vein and providing a stable contact pressure (Figure 1a and 1b).

With some ablation systems such as those used for atrial fibrillation and ventricular tachycardia, there is no method of checking for transmurality so that there is a significant variation in power delivery and duration of power delivery between different operators. As a result of this, there is a significant recurrence rate of arrhythmias, between 30 to 50 percent for atrial fibrillation. For ventricular tachycardia, the recurrence rate is even higher because ventricular tissue is thicker and so it is harder to achieve transmurality.

Studies have shown that when inpatients come back with recurrence of atrial fibrillation, the pulmonary veins typically have reconnected, proving the inability of current ablation systems to achieve transmurality.

Histology studies have shown that a rim of unablated tissue as little as 1.4 mm can lead to reconnection of pulmonary veins and lead to recurrence of atrial fibrillation.

In ablation catheters adapted for renal denervation where the sympathetic nerves on the surface of the renal artery are the targets for ablation, there is currently no endpoint for ablation. As such, the operator is not able to discern when the nerves have been ablated. As such there have been reports of thrombus formation and even dissection of the renal artery, due perhaps to delivering too much power for too long. Accordingly, It would be desirable to provide a system that is configured to interrogate deeper layers of tissue to select out the layer of tissue for ablation and to observe for transmurality with NIR spectroscopy that can provide a constant contact force and also reduce the effect of the beating motion of the heart. To achieve this, diffuse reflectance spectroscopy must be configured to be able to pick up the deeper layers of tissue. Spatially offset diffuse reflectance spectroscopy (SODRS), where there is a spatial offset between the illumination and the receiving optic fibers will allow NIR interrogation of the deeper layers of tissue.

Cui at al have shown that the path of photon travel through tissue is banana shaped so that the depth of tissue interrogated will be approximately half the distance of the spatial offset. Cui, W., Kumar, C. and Chance, B. (1991) Experimental study of migration depth for the photons measured at sample surface. I. Time resolved spectroscopy and imaging. Proc. SPIE Int. Soc. Opt. Eng. 1431, 180±191. Because depth of the muscle layer in the left atrium can range up to a maximum of 5 mm, a spatial offset of 10 mm is required to optically interrogate the deepest layer of muscle in the left atrium. The intimal layer of the renal artery can be up to to 6 or 7 mm thick. Renal denervation targets the sympathetic nerves that lie within the layer of adventitial fat which is another 2 mm thick. Therefore for renal denervation, an offset of 20 mm may be required. In ventricular tachycardia ablation, the tissue to be ablated is thicker. Therefore a bigger spatial offset of 30 to 35 mm between the illuminating and receiving fibers will be required. This can be achieved with a basket with a relatively larger diameter in the larger cavity of the ventricle.

The geometry of standard ablation catheters does not permit a wide spatial offset between illuminating and receiving optic fibers positioned at the distal tip that is required to truly achieve analysis of transmurality for the reason that typically, only between 3 to 4 mm of the distal tip of the catheter is in contact with the muscle tissue. It is difficult to ensure a constant end-on or side-on contact of standard ablation catheters with the tissue surface. Furthermore, the standard systems typically have a spatial offset of 1.7 mm which means that they are only interrogating the superficial tissue layers of 0.4 to 0.8 mm.

## Summary of the Invention

In one aspect, a tissue composition monitoring apparatus, an ablation lesion monitoring method and an ablation lesion determination automated algorithm are disclosed, the algorithm incorporating Arrhenius's model for thermal denaturation kinetics for determining the characteristics of the ablation lesion. The apparatus includes a basket catheter configuration for achieving stability and consistent contact pressure during ablation for collection of optical spectra. The basket catheter incorporates a linear portion which hugs the conical portion of the pulmonary vein during atrial fibrillation ablation and allows a variable spatial offset of between 10 mm to 20 mm between the illuminating optic fiber and the receiving optic fiber.

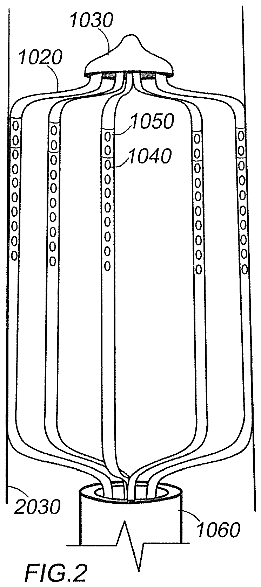

One apparatus is adapted for renal denervation whereby the diameter of the basket is 4 mm and the spatial offset can range from 8 mm to 20 mm due to the fact that the longer horizontal, i.e., more tubular geometry, of the renal artery can allow for a longer linear segment of the basket catheter to abut the inner lumen of the renal artery (Figure 2). The basket catheter is delivered to the target site in a collapsed configuration within a sheath.

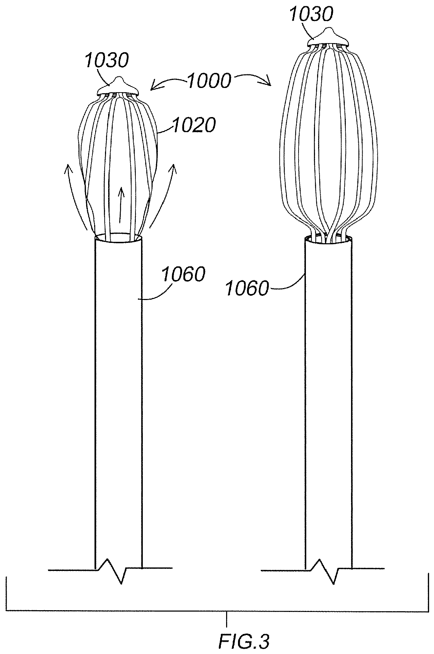

For atrial fibrillation ablation, the sheath is placed within the pulmonary vein under fluoroscopic guidance and the sheath is withdrawn, which allows the basket to assume its preformed shape which will then abut the inside of the pulmonary vein (Figure 3).The basket is slowly withdrawn until the wider part of the basket pops out of the vein and abuts the conical part of the vein. The narrow part of the basket will stay inside the vein and help to stabilize the catheter whereas the wider part will provide a linear stretch of 10 mm of apertures for performing spatially offset diffuse reflectance near infrared spectroscopy.

For renal denervation, the sheath is deflected into the os of the renal artery and the basket catheter is deployed into the renal artery, with the expanded basket catheter abutting the inner lumen of the renal artery. An optical sensor comprises a plurality of splines each containing optical fibers with the illuminating and collecting fibers spatially offset to achieve greater penetration depth.

The apparatus is in the form of a catheter with the distal end comprising a basket configuration. The catheter can be introduced into a person or an animal by means of a sheath and when in the desired organ, for example the heart, the sheath can be directed into the pulmonary vein, the sheath withdrawn and the basket configuration allowed to expand within the vein, thus maintaining stability for optical spectra collection.

The apparatus comprises an energy delivery element for delivering of energy to tissue. The energy delivery element is preferably integrated with the catheter together with optical elements. Tissue characteristics can be determined before energy is applied and during application of energy.

The energy delivery element can be a radiofrequency electrode for applying RF energy. However, other types of energy may be used, for example laser ablation or cryoablation.

The optical sensor element comprises a basket catheter with a plurality of splines containing a plurality of optic fibers. One of the optic fibers is for illuminating and the other optic fiber is for collection. The illuminating fibers and the optic fibers are spatially offset from each other to achieve greater depth penetration in order to assess the characteristics of the tissue, for example transmurality of the ablation lesion. The illuminating optic fibers are connected to a light source like a laser and the optic fibers guide the light to the tissue. The collection fibers are preferably connected to a spectrophotometer for generating optic spectra of the tissue wherein the apparatus is adapted to determine the characteristics of the tissue, for example transmurality of the ablation lesion depending on the characteristics of the optic spectra.

The apparatus can be adapted to determine the transmurality of the ablation lesion from the optical sensing data. The optical sensing data are indicative of the absorption and scattering properties of the tissue. The apparatus can therefore be adapted to assess the transmurality of an ablation lesion and/or the composition of the tissue based on the optical sensing data.

In a preferred case, the apparatus comprises computer memory in which the optical spectra are stored, which are assigned to ablated tissue with 100% transmurality.

The apparatus is also preferably adapted to compare the stored (known) spectra with actually measured spectra in order to differentiate between muscle and fibrosis (collagen), between fat and muscle, between fat and nervous tissue, in addition to the transmurality of ablated tissue.

The apparatus can comprise a memory in which optical spectra are stored which are assigned to different types of tissue, for example, fat, muscle, collagen, nerve and water. The apparatus can be adapted to determine the type of tissue by comparing the stored spectra with the actually measured optical spectra.

Energy can be applied continuously for example, in a circle around the pulmonary veins in atrial fibrillation ablation. The apparatus can be adapted to determine whether ablation has been carried out continuously along the circle depending on the optical sensing data. The basket configuration is adapted to deliver energy at multiple ablation points simultaneously. This can achieve a one-shot ablation where due to the density of the splines, only one ablation is needed for a continuous circle around the pulmonary veins, or an ablation where two or three shots are required and achieved by rotating the basket clockwise and counterclockwise from the initial ablation location. Two or three shots may be required in a case where the number of the splines is kept for example to 8 splines to reduce the stiffness of the basket (Figure 1).

For renal denervation, the object is not to achieve a circumferential ablation around the renal artery in order to avoid stenosis of the artery. Instead, the object is to cause ablation lesions in a spiral fashion along the long axis of the renal artery. Ablation can be carried out selectively at ablation electrode 1 and 5 and then the catheter can be moved distally. Ablation is then carried out at electrodes 2 and 7 and the catheter is then moved more distally, and ablation is carried out at electrodes 3 and 8.

Perfusion by fluid can be achieved through the apertures on the splines or through a central shaft.

The present apparatus is adapted to control the delivery of energy depending on the rate of change of the optical properties of the tissue. The rate of change of the optical properties of the tissue being ablated can be used to track the process of tissue denaturation. The rate of change of the optic properties of tissue can be fit to a curve with the exponential function I - exp(-t/r), which is modeled on Arrhenius equation for thermal denaturation, thus allowing the extraction of the rate constant from the slope of the curve at the point where 63 percent of the tissue is denatured. The point at which the rate of change of optical properties is zero is associated with 100 % transmurality. The rate constants for the optimal thermal denaturation reaction where 100% transmurality is achieved with a temperature of 60 degrees Celsius for a duration of between 30 to 60 seconds are determined in in vivo and ex vivo experiments using nitroblue tetrazolium (NBT) staining of tissue to determine viability and hence transmurality and are stored in the memory. Thus as energy is delivered, the rate of change of optical sensing data can be used to generate a graph and the graph generated can be compared to a stored graph and the rate of the change (slope) and energy adjusted or titrated to achieve a desired rate or graph or slope.

A display unit can be provided for showing optical data and the percentage of transmurality and/or composition of the tissue.

A computer program can be provided which includes a program code means for causing a computer to carry out the steps of an ablation lesion assessment method when the computer program is run on the computer.

**Brief Description of the Drawings**

Figure 1a and 1b depict a catheter with a cone shaped basket configuration at the distal end adapted for atrial fibrillation ablation;

Figure 2 depicts a catheter with a tubular basket configuration at the distal end adapted for renal denervation;

Figure 3 shows a basket catheter delivered to the target site in a collapsed configuration within a sheath. The sheath is placed within the vessel (pulmonary vein or renal artery) under fluoroscopic guidance and the sheath is withdrawn, which allows the basket to assume its preformed shape which will then abut the inside of the vessel;

Figure 4 shows a basket configuration with alternate shapes;

Figure 5 is an end-on view of a basket;

Figures 6 and 7 shows a basket closely hugging the contours of the vein;

Figure 8 depicts the structure of each nitinol spline tubing in the basket configuration for atrial fibrillation ablation;

Figure 9 depicts the structure of each nitinol spline tubing in the basket configuration for renal denervation;

Figure 10 shows a basket catheter used for renal denervation where the shape of the basket is rectangular or cylindrical, to conform to the tubular structure of the renal artery;

Figure 11 shows a copper wire (50 to 100 micrometers in diameter) positioned on the inside wall of the spline to connect to the electrode;

Figure 12 shows that within a hollow tube of each of the arms or splines, optic fibers are placed on top of each other;

Figure 13 depicts an example of atrial fibrillation ablation wherein an illuminating optic fiber is situated below the receiving optic fiber and is located proximal to the receiving fiber;

Figure 14 depicts an example of renal denervation wherein an illuminating optic fiber is situated above the receiving fiber and is distal to the illuminating fiber;

Figure 15 is a schematic view depicting components of a system;

Figure 19 shows an algorithm for atrial fibrillation ablation;

Figure 20 shows an algorithm for renal denervation;

## Detailed Description of the Preferred Embodiments

Reference Listing

    **1000** catheter
    **1010** optical sensor
    **1020** spline/arm
    **1024** photon path
    **1025** optic fiber
    **1027** receiving fiber
    **1028** illuminating fiber
    **1029** mirror
    **1030** cap
    **1040** aperture
    **1050** electrode
    **1051** wire to electrodes
    **1060** sheath
    **1070** basket
    **1080** MEMS gyroscope
    **2000** pulmonary vein
    **2010** opening pulmonary vein
    **2020** renal artery
    **2030** opening renal artery
    **3000** ablated tissue

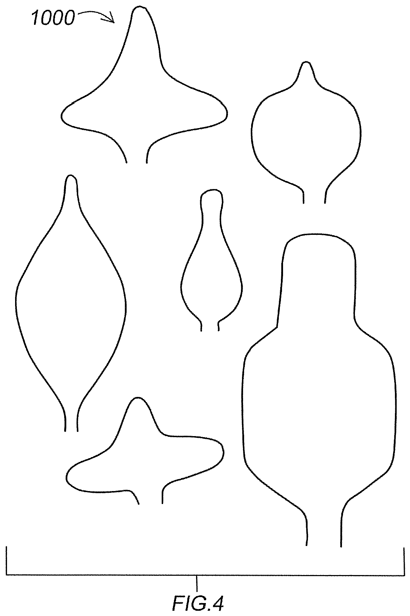

The present apparatus includes a catheter including optical sensors for generating optical sensing data that are indicative of the optical property of the tissue. Figure 1 shows a preferred apparatus for determining the properties of a tissue. The distal end of catheter **1000** expands into a basket **1070** configuration with a plurality of arms or splines **1020.** By way of examples shown in figure 4, the basket can have a plurality of shapes. In a preferred case, the basket has 8 splines or arms.

Figure 2 shows the distal end of the catheter with the basket. The distal end of each of arms **1020** is attached to a soft, non-traumatic cap **1030** and is preferably made of PEBAX which has been doped with a radiopaque material like barium sulfate (Figure 1a and 1b).

The basket configuration can include wires, ribbons, cables and struts and can be constructed from either metals, non metals or combinations of both. The basket configuration can be coated with Duraflo. Elements of the basket configuration can be made of one or more materials, including both metals and non metals. Typical alloys chosen for basket construction include nitinol, stainless steel, Elgiloy, other alloys or any combinations thereof. The distal basket of the catheter has a preformed flexible shape which is designed to conform to the vessel in which the basket is placed. For atrial fibrillation ablation, the basket is shaped like a cone to abut the pulmonary vein.

A catheter **1000** includes a plurality of tubular components that can be steered by including a controllable pull wire at or near the distal end. Specifically, the catheter of the present apparatus can include an integral steering means, similar to existing catheters, such as a plurality of pull wires attached near a distal portion of the catheter and operably attached to a lever, knob or other control integral to a handle of the catheter. The steering mechanism is used to deflect the basket (used in conjunction with an outer sheath **1060** which may or may not be steerable) and distal end of the catheter into the left and right pulmonary veins of the left atrium. The integral catheter steering means can be used with a steerable transeptal sheath. A plurality of pull wires can be mounted 90 degrees apart at different locations in a distal portion of the catheter to provide multi-axis, precision controlled steering.

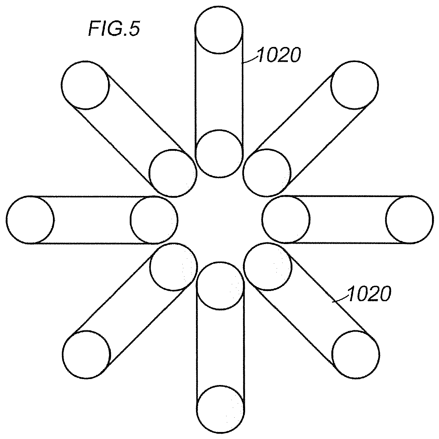

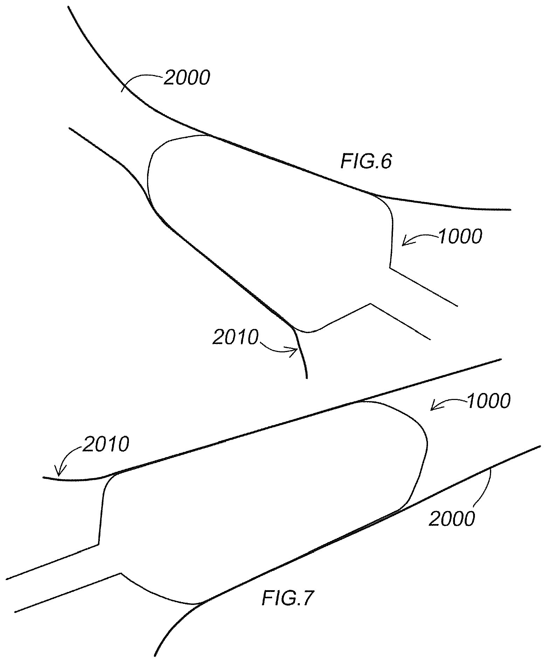

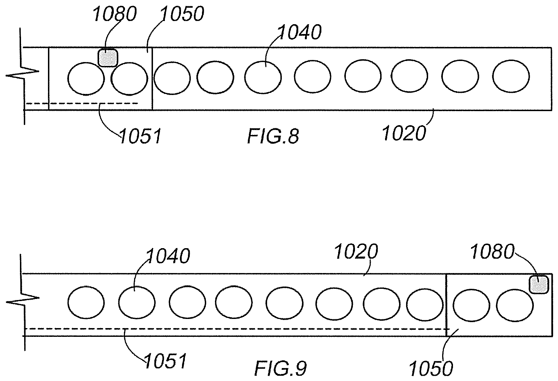

Basket **1070** is introduced into the pulmonary vein via the deflectable sheath **1060** (for example the Agilis sheath, St Jude). The sheath is then withdrawn and the basket is allowed to expand fully to its preformed shape. Figure 5 shows the end-on view of the basket. As seen in Figures 6 and 7, the basket will closely hug the contours of the vein. The basket is adapted to be deformable such that when the outer steerable sheath is withdrawn, the basket will expand to its maximum diameter to abut and press tightly against the conical portion of the pulmonary vein which extends from the os of the vein to approximately 20 mm into the vein. The basket is adapted with this linear segment of the basket designed with a series of drilled apertures **1040** measuring 300 micrometers each and separated by 760 micrometers enclosing optic fibers 1025 for performing spatial offset diffuse reflectance spectroscopy (Figures 8 and 9). Ensuring close contact between basket and tissue and critically maintaining stability and constant contact pressure is important for optical sensing as motion artifacts and different contact pressure can affect optical sensing data.

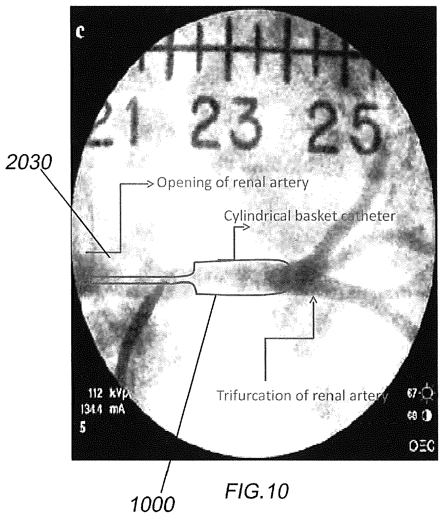

In a preferred version of a basket catheter used for renal denervation, the shape of the basket is rectangular, to conform to the tubular structure of the renal artery. **2020** (Figure 10). For renal denervation, the size of the basket typically ranges from 2 mm to 6mm in diameter and it has a longer flat, linear segment (figure 2). The size of the basket can range from 2 mm to 70 mm in diameter. The length of each of the splines **1020** may range from 45 mm to 70 mm from the distal end of the basket, where the spline attaches to the soft atraumatic cap **1030** to the proximal end. In a preferred case, 10 holes are drilled into each arm or spline with the spacing of each of the holes being 760 micrometers from each other. In this preferred embodiment, each of the splines of the basket contain 10 apertures **1040** for allowing light from the illuminating optic fiber to illuminate tissue and the receiving optic fiber to receive light from the tissue. The diameter of the holes is 300 micrometer. The holes can have a 300 micrometer ball lens (as manufactured by DSI) to collimate and focus the returning light from the tissue to the receiving optic fiber wherein the ball lens is bonded to the hole by epoxy.

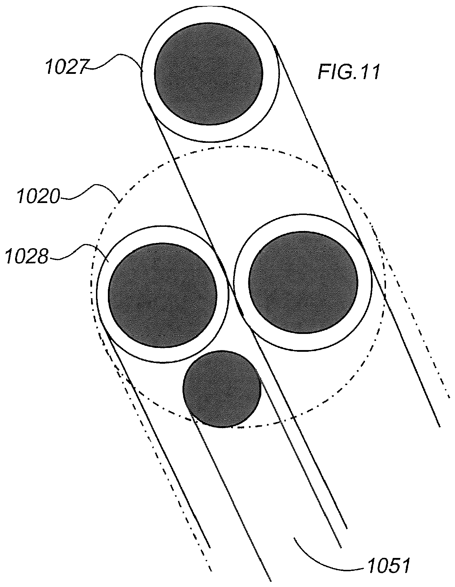

An energy application element is integrated into the catheter **1000**' wherein the energy application element consists of electrodes **1050** for applying energy to the inner wall of the heart. The electrodes are connected to an energy source via electrical connections for providing electrical energy to the inner wall of the heart. The electrical connections are preferably wires located within the catheter. The 2 distal-most holes are enclosed by an electrode. Electrodes of a platinum-iridium alloy are mounted to the arms with cyanoacrylate or other adhesive beads. Each electrode carries a thermocouple, preferably a copper-constantan wire junction welded to the internal surface of the electrode. Each electrode, and any included thermocouple is attached to one or more wires to form a wire bundle, which travels proximally and is attached to an electrical port on the proximal end of the ablation catheter. A copper wire (50 to 100 micrometers in diameter) runs on the inside wall of the spline to connect to the electrode. (Figure 11). Each of the electrodes is attached via connecting wires and one or more connectors such as a conduit to an energy delivery apparatus, preferably an RF energy delivery unit, which is also attached to a patch electrode but preferably to a conductive pad attached to the back of the patient. The energy delivery unit is configured to deliver RF energy in monopolar, bipolar or combination monopolar-bipolar energy delivery modes, simultaneously or sequentially with or without off or no energy delivered time duration.

In a preferred case, the energy delivery unit is configured to provide electrical mapping of the tissue that is contacted by one or more electrodes integral to the basket. Alternatively, a separate mapping unit such as a MEMS gyroscope **1080** may be used, preferably attached to the catheter simultaneously with attachment to the energy delivery unit (Figures 8 and 9), in which case, copper wire **1081** also runs to the MEMS gyroscope.

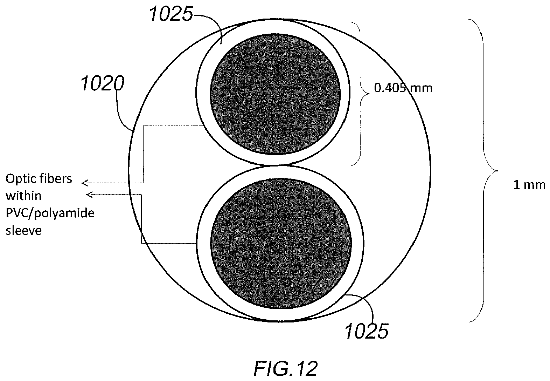

The catheter has an optical sensor **1010** integrated into it, the sensor possessing a light emitting means and light receiving means. The optical sensor is adapted to generate optical sensing data depending on the received light. Light emitting and receiving means of the optical sensor comprise optic fibers. Illuminating optic fibers **1028** are connected to a light source such as a laser and the optic fibers guide the light to the tissue. The collection fibers **1027** are preferably connected to a spectrophotometer for generating optic spectra of the tissue, wherein the apparatus is adapted to determine the characteristics of the tissue, for example transmurality of the ablation lesion depending on the characteristics of the optic spectra. A plurality of optic fibers are enclosed within each of the arms or splines **1020**. One manufacturer of suitable optic fibers is Polymicro, Arizona. The optic fibers are multimode fibers and are 200 micrometers in diameter and have a numerical aperture of 0.22. Within a hollow tube of each of the arms or splines, the optic fibers are placed on top of each other (Figure 12). The optic fibers can be covered or coated with PVC or polyamide to prevent crosstalk between the fibers.

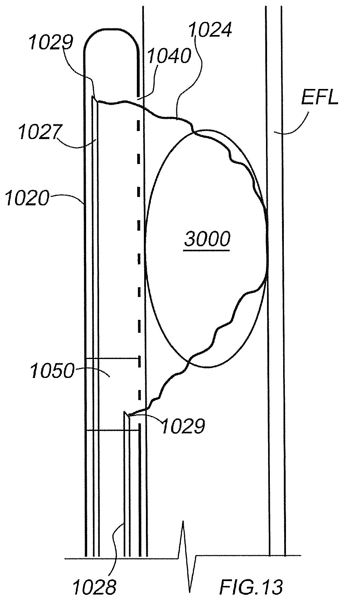

In cases of atrial fibrillation ablation, for example, the illuminating optic fiber is situated below the receiving optic fiber and is located proximal to the receiving fiber. The illuminating optic fiber can be movable/ translatable or fixed through application of cement (e.g., Norland 93, Norland Products) to the inner wall of the hollow tube of the arm or spline (Figure 13).

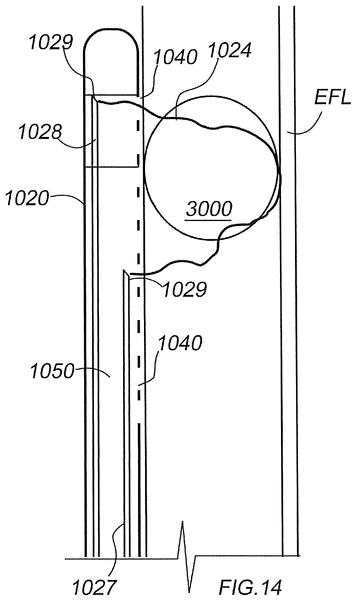

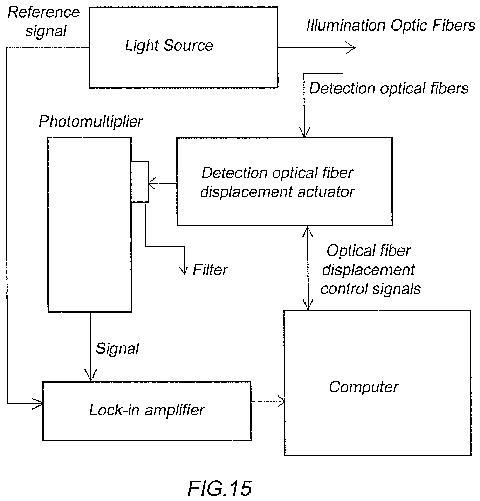

For renal denervation, the illuminating optic fiber is situated above the receiving fiber and is distal to the illuminating fiber (Figure 14). The receiving optic fiber is not fixed but movable, and can be adapted to achieve a linear and rotational scan of the tissue by translating the optic fiber through a range of a linear distance between 2 to 20 mm and a rotation of between 10 to 60 degrees, respectively. The driving force for the scan is provided by a linear/rotational galvanometer (Figure 15). The translation provided by the linear galvanometer is transmitted to the tip by a polytetrafluoroethylene coated polyimide tube (Microlumen, Tampa, Florida) containing the receiving optical fibers (Figure 11).

In another case, the galvanometer can also rotate the receiving and illuminating optic fibers to scan unablated areas on either side of the ablated area to provide an assessment of the area ablated. Both the illuminating and receiving optical fibers are adapted to illuminate the inner wall of the heart in a sideward looking direction. Light is coupled out from an optical fiber at a certain angle; e.g., 43 degrees, by polishing a slanted distal end on the respective optical fiber and by coating the surface at the slanted end with a metallic layer. Each of the receiving and illuminating optic fibers comprises a core and a cladding. On the polished slanted end surface of the optical fiber, a metallic coating is provided for forming a mirror **1029**. The metallic coating can be, for example, a layer of approximately 100 nm of silver on a chromium adhesion promoter layer of approximately 5 nm. The light from the illuminating optic fiber is guided along the light path such that it is reflected by the mirror in a sideward looking direction. Light travelling back from the tissue at the receiving optic fiber similarly passes through the apertures into the distal end of the fiber and is reflected by the mirror back along the axis of the optic fiber. The returning light from the tissue may be collimated by a ball lens into the slanted end of the receiving optic fiber. The apertures can also be adapted for providing irrigation.

The present apparatus may comprise an irrigation control unit being connected with the irrigation openings via an irrigation tube in order to allow the user to control irrigation of the cardiac tissue.

Setting appropriate end points for ablation

1) Atrial fibrillation ablation

As tissue is undergoing heat denaturation for example by ablation, optical properties change. Beauvoit et al reported that the mitochondrial compartment is the primary cause of light scattering. Thomsen et al. (US), hereinafter Thomsen, observed disruption of mitochondria into coarse granules, and discerned small aggregates resulting from denaturation of fibrillar contractile proteins and other cytoplasmic constituents. Based on their observations, Thomsen proposed that these thermally induced morphological changes are the physical cause of the increased rat myocardial tissue scattering observed with thermal coagulation.

By monitoring changes in these scattering optical properties, one can monitor the progress of the ablation. Optical properties commonly used to monitor the progress of ablation can be scattering, absorption or reflectance. Reflectance can be an effective property in order to monitor the progress of ablation. Reflectance however, is a composite of the changes in absorption and scattering that is occurring as the ablation process continues. Monitoring the progress of ablation can also be confounded by the differing absorption properties of the different layers of tissues that are present in the tissue to be ablated. Furthermore, as the ablation process continues, blood leakage, tissue edema and tissue shrinkage can lead to dynamic changes in scattering and absorption properties which can confound the monitoring process. For example, tissue swelling and edema can occur after 500 seconds. One must therefore account for these aforementioned factors in order to be able to use reflectance to monitor the ablation process accurately. The reflectance signal is invariant to increased absorption at 800 nm. We can therefore probe ablated tissue based on measurement of the ratio between the reflectance signal at two wavelengths, for example one wavelength around the wavelength of interest (ranging from 400-2500 nm) and one around 800 nm. Normalized reflectance values are therefore obtained by obtaining a ratio of the reflectance at a particular wavelength to the reflectance at 800 nm. This ratio will allow the cancelling out of absorbance. Normalized reflectance will be used to monitor the ablation process in real time by tracking the rate of change of the normalized reflectance.

As ablation is being carried out, conductive heating and thermal diffusion through tissue leads to temperature elevation and the tissue temperature rises. This leads to the changes in the size and/or location of the optical scattering centers like the mitochondria due to disruption of mitochondria into coarse granules, and denaturation of fibrillar contractile proteins and other cytoplasmic constituents resulting in the formation of small aggregates leading to an increase in the normalized reflectance. When ablation is started, there may be a lag phase of between 1 to 4 seconds (which may represent the time it takes for conductive heating and thermal diffusion through tissue) before there is a rise in the normalized reflectance. A steady rise in normalized reflectance is followed by a plateau where there is no change in the normalized reflectance, at which point the tissue is considered 100% denatured at that depth. Changes in normalized reflectance during the early time course of the ablation are fitted to the curve of the exponential function I - exp(-t/r).

By modeling the above reaction on the Arrhenius equation for thermal denaturation, one can obtain the rate constant for the reaction which is equivalent to the slope of the curve at the point where 63 % of the tissue is denatured. Referring to Fig. A, in the reaction defined by the exponential function I - exp(-t/r), r is the rate constant of the denaturation reaction and is the value of the normalized reflectance when 63 percent of the tissue is denatured.

The Arrhenius equation for thermal damage is given by:

$$\Omega(\tau) = \int_0^\tau A\, e^{\left[\frac{-E_a}{R\,T(t)}\right]}\, dt$$

where $\Omega(t)$ is the damage integral and is the logarithm of the ratio of the original concentration of native tissue to the remaining native tissue state at time t, A is a frequency factor ($s^{-1}$), $\tau$ is the total heating time (s), $E_a$ an activation energy barrier ($J.mole^{-1}$), R the universal gas constant, 8.3143 ($J.mole^{-1}.K^{-1}$) and T the absolute temperature (K). The damage integral is therefore an indication of the percentage of proteins having undergone denaturation. The damage integral is given by the rate of change of normalized reflectance. There is a critical temperature for which the damage integral is 1, that is 100 % of tissue would have undergone denaturation for a given duration. For myocardial tissue, this critical temperature is 60 degrees Celsius. Accordingly, the Arrhenius equation predicts that for a gives temperature, 100 % of tissue will be denatured for a given duration of exposure. For a series of lesions created at different temperatures but resulting in equivalent damage, a linear fit would yield AE/R as the slope, and - ln(A) as the intercept.

The Arrhenius model predictions for tissue damage is then compared to histological results, for example, nitroblue tetrazolium staining of ablated tissue as a marker of transmurality of the lesion, where 100% denaturation is equivalent to 100% transmurality.

Ex vivo and in vivo experiments are performed where the rate constant associated with a range of tissue temperature from 45 to 75 degrees Celsius (as determined by a thermocouple embedded in the tissue) and 100 % transmurality (as determined by histology using NBT to assess for non viability of tissue) will be determined and stored in the memory.

The optimal rate constant associated with a tissue temperature of 60 degrees Celsius which will ensure 100% transmurality (as determined with histology using NBT to assess for non viability of tissue) within an ablation time of between 10 to 60 seconds will be determined from the ex vivo and in vitro experiments and will be stored in the memory. At the point where 100% tissue denaturation occurs, there is no further change in the reflectance properties of tissue observable as a plateau (the rate of change of normalized reflectance is zero). Therefore, by monitoring the ablation process in real time, one can determine precisely the moment when the tissue is denatured.

The point at which 100% tissue denaturation occurs leading to the plateau is time sensitive. There is a late phase after about 300 to 500 seconds where edema, blood leakage and tissue shrinkage will occur which can lead to changes in the normalized reflectance. Immediately at the time point where the plateau is reached, the illuminating and receiving optic fibers are scanned rotationally by the galvanometer from side to side, to determine the area of the ablated region at the surface. For determining the optical spectra, the optical sensor can illuminate the inner wall of the heart with light having different wavelengths, wherein the optical spectra are generated by using the spectrometer. Alternatively, the optical sensor can be adapted to illuminate the inner wall of the heart at different wavelengths temporally consecutively such that the optical fibers for receiving light from the inner wall of the heart receive light from substantially only one wavelength. The wavelength of interest may range from 400 nm to 2500 nm. For example, a wavelength of 2060 nm can be selected, wherein a shift to a lower wavelength of 2056 nm corresponding to protein unfolding and protein denaturation from thermal energy application can be used to discriminate between ablated and non ablated muscle tissue. The receiving optic fiber is rotated until the NIR spectra for non ablated tissue are discerned, whereby the angle of rotation with the distance travelled gives an indication of the area ablated. Information obtained in the foregoing steps can be integrated with depth information to give a 3D representation of the volume of ablated tissue, and is compared to histological and also spectroscopic assessments of the volume of NBT dye staining.

By incorporating the measurement of the changes in normalized reflectance with respect to time into the Arrhenius equation for thermal damage, the present method offers the advantage that the plateau where the rate of change of normalized reflectance is zero can be registered sensitively at an early time frame/window (20 to 80 seconds) before edema, blood leakage and tissue shrinkage sets in (300 to 500 seconds) and therefore avoids the confounding influence of these factors on tissue optical properties.

Ex vivo and in vivo experiments determine the lag phase r which is the rate constant (or slope) for that reaction at a particular temperature and with a particular time to plateau which will ensure 100% transmurality as based on NBT staining of tissue. Accordingly, there is an optimal lag phase, slope and time to plateau that we require to get complete lesion before tissue edema sets in and prevents the achievement of an effective ablation lesion. Stored rate constants of various energy and power settings (and the corresponding temperature) correlating with different times to plateaus, and the lag phase associated with different pathological assessments showing different degrees of transmurality will be stored in the memory and used to guide ablation.

In cases where the operator is using too low a power setting, there will be a significant deviation from the Arrhenius model and the algorithm can operate to shut off the power to prevent the operator from causing an ineffective lesion or the algorithm can increase the power to get the operator back onto the curve (the right rate constant) and back onto the right track.

Based on the data accumulated in ex vivo and in vivo experiments, the present algorithm will be able to take the operator's power settings, lag phase and rate constant, compare them to the data stored in memory and allow the operator to calculate the percentage of denatured tissue in real time; i.e.,10%, 20 % 50% and tell the operator when he is done. This can be displayed on a user interface display unit. Because the measurements of normalized reflectance are performed as a function of time and depth, an M mode image can be generated that will provide a visualization of the thermal process. Using data from ex vivo and in vivo experiments, the depth information obtained from the spatially offset diffuse reflectance is compared against the histological data. This data is then integrated with data about the area of the ablation lesion at the surface to generate a 3D representation of the lesion which can be generated in real time.

The method is also adapted to translate the receiving optic fiber as the ablation process is occurring, wherein the initial position of the receiving optic fiber before ablation is carried out is close to the illuminating fiber. As the ablation starts, the receiving optic fiber is translated away from the illuminating optic fiber. The distance between the illuminating and receiving optic fiber corresponds to the depth at which the photons have traveled and represents the depth of the muscle layer from which the optical sensor is relaying optical sensing data.

The galvanometer is programmed to track the duration of the ablation process (according to the rate constant and the time to the plateau data from in vivo and ex vivo experiments,) in the position for between 20-60 seconds until the plateau is obtained signifying that the muscle layer is denatured at this depth. By performing these measurements as a function of time and depth, M-mode images that provide a visualization of the thermal therapy process can be generated. The depth-localized intensity of the signal magnitude fluctuations is mapped according to a color bar. The maximum amplitude of the reflectance signal is plotted against time in an M-mode image. The M-mode image is formed by mapping the reflectance signals to the indicated color space.

*Determination of the scattering and absorption coefficients from spatially offset diffuse reflectance spectroscopy*

The present method is further adapted to determine the kind of tissue or the composition of tissue. Optical spectra assigned to different types of tissues for example, fat, nerve, muscle, collagen, water are stored in the memory, wherein the present method is adapted to determine the respective kind of tissue or the composition of tissue by comparing the stored optical spectra with actually measured optical spectra. For determining the optical spectra, the optical sensor can illuminate the inner wall of the heart with light having different wavelengths, wherein the optical spectra are generated by using the spectrometer.

Alternatively, as described in the foregoing exemplary case above under the section "Setting appropriate end points for ablation", the optical sensor can be adapted to illuminate the inner wall of the heart at different wavelengths temporally consecutively such that the optical fibers for receiving light from the inner wall of the heart receive light from substantially only one wavelength. In this situation, a spectrometer may not be needed but a photodetector that is sensitive to the respective wavelength. Therefore, the output of the optical fibers, which have received the light from the inner wall of the heart can be detected by a photodiode for generating a detector signal and the detector signal can be processed in accordance with an algorithm as will be described below.

The present method uses spatially offset diffuse reflectance spectroscopy to collect reflectance spectra. It is advantageous to extract scattering and absorption coefficients from reflectance data obtained by SODRS. In order to extract absorption and scattering properties from reflectance measurements, spatially resolved intensities need to be obtained. The present method is adapted to measure the diffusely reflected light intensity at the surface of the tissue at different distances from the small illuminated surface spot. For tissue, the separation distances are in the range of between 2 to 20 mm. Therefore, the optical properties are derived from reflectance profiles measured at 10 source detector separated locations. The present method uses a scanning approach with a side deflected illumination fiber and collection fiber that can be translated axially. The excitation light enters the probe via an optical fiber and is focused on the tissue surface near the end of the probe with the aluminized mirror surface on the polished angled surface of the distal end of the optic fiber. The intensity of the diffusely reflected light at the surface at different distances from the excitation spot is probed by displacing the movable optical fiber along the probe in such a way as to measure the light passing through the holes one after the other (counting the number of maxima in the measured diffuse reflectance curve). This automatically gives the radial distance from the illumination spot along the surface, as the position of each hole is known. The fiber of the detection assembly then guides the light to a photomultiplier, the signal of which is amplified using a lock-in technique. A monochromator is inserted between the fiber end and photomultiplier to avoid possibly important contributions from autofluorescence. The logarithm of the observed signal I (arbitrary units) is plotted against the distance along the surface between excitation source and probing point. This data is then fitted to an analytic expression, which is based on diffusion theory. From the radial dependence of the diffusely reflected light intensity at the surface of the organ concerned, the tissue optical parameters may be extracted using the Groenhuis model.

The present method can be adapted to use the algorithm to extract the tissue optical parameters, the scattering and absorption coefficients as disclosed in "Clinical optical dose measurement for PDT : Invasive and non-invasive techniques" by R Bays et al., SPIE Vol. 1525 Future Trends in Biomedical Applications of Lasers (1991), pages 397 to 408.

The present method, using the algorithm described above can therefore determine the scattering coefficients and the absorption coefficients of different tissue chromophores like fat, water, muscle, collagen and nerves.

The present method can also be adapted to discriminate differences in optical spectra by making use of a principal components analysis as disclosed in "Partial Least-Squares Methods for Spectral Analyses. 1. Relation to Other Quantitative Calibration Methods and the Extraction of Qualitative Information by David M Haaland al.,Analytical Chemistry, vol. 60, no.11 pages 1193to 1202, June 1988. The principal component analysis allows classification of differences in optical spectra and, thus allows discrimination between tissues, in particular, between fat and muscle, between muscle and collagen, between fat and nerves and between ablated and non ablated tissue.

The present method can also be adapted to discriminate between areas of heart muscle tissue infiltrated with fibrosis and areas with no fibrosis. In this case, a bigger sized basket (for example 50 mm in diameter) is placed in the left atrium, so that the basket approximates the endocardial surface of the left atrium.

The receiving optic fiber is scanned along the length of the spline in an axial direction and also rotationally in a side to side manner. As the receiving optic fiber is scanned along the surface of the tissue, it is possible to distinguish for example, between areas of fibrosis (collagen) and muscle, fat and muscle, nerve and fat, muscle and fat.

The present method is adapted to localize the depth of the fibrosis, fat, nerve and muscle with the algorithm described above that is adapted for spatially offset diffuse reflectance spectroscopy. The areas and location of interest, for example fibrosis, fat, and nerve can be tagged with the localization unit that is adapted to provide x, y and z coordinates of the area of interest, with a color scale denoting the concentration of fibrosis correlated with the intensity of the optical parameter (for example, reflectance, scattering or absorption coefficient).

**Before ablation is started, the thickness of the muscle tissue layer to be ablated is determined**

As mentioned previously, the present method can be adapted to distinguish between fat and muscle to determine the depth at which ablation should be carried out by comparing an actually measured optical spectrum with stored optical spectra which are assigned to fat or muscle tissue.

In atrial fibrillation ablation, the adventitial fat layer represents the boundary beyond which the ablation should not be carried out, in order to avoid damage to the esophagus which lies beyond the adventitial fat layer of the left atrium.

The present method can take advantage of the different optical properties of fat and muscle to limit the ablation before the fat layer to prevent collateral damage. The NIR spectra for fat tissue and for muscle tissue and for nerves are stored in the memory. In order to compare an actually measured optical spectrum with stored optical spectra, the present method is preferentially adapted to use a similarity measure like a correlation or a sum of squared differences.

At the beginning of the ablation procedure, the illumination optic fiber is fired and the receiving optic fiber is scanned along the tissue to probe the deepest depth of the tissue until it registers the NIR signature of fat. This represents the deepest adventitial fat beyond which the ablation should not proceed. This is also the layer where critical structures like the phrenic nerve, adjacent to the right superior pulmonary vein are located. If the initial scanning process registers the NIR signature of nerve over the location of the right superior pulmonary vein, an alternative site may be targeted for ablation to prevent injury to the phrenic nerve. The depth at which the fatty adventitial layer is recorded is for example 5 mm. The spatial offset is then adjusted until the NIR signature of muscle is recorded. The muscle layer of the left atrium lies superficial to the fatty adventitial layer, so the depth of this muscle layer will be for example 4 mm. Ablation is then carried out. As discussed above, as the ablation is carried out, a change in reflectance occurs. This change in reflectance will be normalized to get a ratio which will cancel out the effects of absorbance. This change in reflectance with respect to time can be fit to an exponential function which is modeled on the Arrhenius equation for thermal denaturation. The Arrhenius equation can predict a 100% transmurality (ie denatured tissue) for a particular tissue temperature (for example 60 degrees Celsius) held for a particular duration (for example 20 seconds). This particular set of conditions is associated with a thermal denaturation exponential curve with a rate constant that is given by the slope of the curve whereby 63% of the tissue has been denatured.

Ex vivo and in vitro experiments are performed with a thermocouple embedded into the target tissue to record the tissue temperature during ablation with a series of tissues temperatures recorded for different power settings and with the tissues stained with NBT (or TTC) to determine the amount of viable tissue and therefore the extent of transmurality (Figure 18). Thus the exponential curve with the rate constant associated with a tissue temperature of 60 degrees Celsius and 100 % transmurality and the duration required to achieve 100% transmurality is stored in the memory.

During an ablation procedure, an automated algorithm will reference this stored value with the current parameters of the thermal denaturation of the ablation process and the power can be adjusted or titrated to approximate the stored value. When the exponential curve reaches a plateau, signifying 100% denaturation, the ablation process is terminated to avoid carrying the ablation process beyond into the adventitial fat layer and causing collateral damage. The following represents a preferred algorithm for atrial fibrillation ablation: Transeptal catherization → Steerable sheath guided into pulmonary vein → Steerable sheath withdrawn → Basket expands to preformed shape → Basket withdrawn until it abuts tightly against the opening of the pulmonary vein with the radiopaque markers on the electrode signifying the desired position of the electrode at the os → Illuminations optic fiber is fired → Receiving optic fiber is translated by galvanometer → NIR spectra are compared with NIR spectra of fat stored in memory → Epicardial fat layer identified → Further scanning and /or rotation of the basket is performed until NIR for nerve is identified → Ablation is performed → Rate of change of normalized reflectance is followed as an exponential curve and the lag phase, and the slope of the curve of the current reaction are compared against the reaction where 100% transmurality was obtained at a temperature of 60 degrees Celsius over a duration of 40 seconds → If the slope is less than the one stored in memory, the computer algorithm then commands the energy delivery unit to increase the power until the slope matches the one for the ideal reaction rate stored in the memory.

Renal denervation

Referring to **fig. 20,** with current ablation devices for carrying out renal denervation, there is no end point to serve as a target for ablation. This can lead to the application of too much power for too long a duration, leading to collateral damage. Using OCT, investigators have recorded thrombus formation and even dissection of the renal artery. The present method can provide an endpoint for ablation for locating the depth where the renal sympathetic nerves are located using the NIR signature for nerves to track it. Once the depth of the nerves is located, for example at 4mm, the ablation is carried out.

The rate constant for the thermal denaturation of nervous tissue is determined experimentally and used to guide the ablation process as described in the atrial fibrillation process. The following represents a preferred algorithm for renal denervation: Femoral artery access → Steerable sheath guided into renal artery with fluoroscopy → Steerable sheath withdrawn → Basket expands to preformed shape → Basket withdrawn until it abuts tightly against the renal artery with the radiopaque markers on the electrode signifying the desired position of the electrode just before the trifurcation of the renal artery → Illumination optic fiber is fired → Receiving optic is translated by galvanometer → NIR spectra are compared with NIR spectra of fat stored in memory → Fat layer identified → Spatial offset at which this occurs is noted → Spatial offset adjusted to a lower offset until a more superficial layer of muscle just adjacent to the fat layer is identified → Ablation is perfomed → Rate of change of normalized reflectance is followed as an exponential curve and the lag phase, and the slope of the curve of the current reaction are compared to the reaction where 100% transmurality was obtained at a temperature of 60 degrees Celsius over a duration of 40 seconds → If the slope is less than the one stored in memory, the computer algorithm then commands the energy delivery unit to increase the power until the slope matches the one for the ideal reaction rate stored in the memory.

Ventricular tachycardia ablation

For ventricular tachycardia ablation, the tissue to be ablated is thicker. Therefore a bigger spatial offset of 30 to 35 mm between the illuminating and receiving fibers will be required. This can be achieved with a basket with a bigger diameter in the larger cavity of the ventricle. The optical sensor may further be adapted to perform transmission spectroscopy by utilizing a plurality of basket catheters, wherein one basket catheter is placed in the pericardial space adjacent to a basket in the ventricular cavity and wherein the optic fibers of the basket in the pericardial space are adapted to receive light from the illuminating fibers from the basket in the ventricular cavity.

Adaptation for Transmission Spectroscope

The optical sensor may be adapted to perform transmission spectroscopy by utilizing a plurality of basket catheters, wherein one basket catheter is placed in an adjacent vein, for example the coronary sinus vein to the left pulmonary veins and wherein the optic fibers of the basket in the coronary sinus are adapted to receive light from the illuminating fibers from the basket in the left pulmonary veins.

The optical sensor may further be adapted to perform transmission spectroscopy by utilizing a plurality of basket catheters, wherein one basket catheter is placed in an adjacent vein, for example the superior vena cava to the right pulmonary veins and wherein the optic fibers of the basket in the superior vena cava are adapted to receive light from the illuminating fibers from the basket in the right pulmonary veins.

The optical sensor may further be adapted to perform transmission spectroscopy by utilizing a plurality of basket catheters, wherein one basket catheter is placed in the pericardial space adjacent to a basket in the ventricular cavity and wherein the optic fibers of the basket in the pericardial space are adapted to receive light from the illuminating fibers from the basket in the ventricular cavity.

**4D electroanatomic mapping system**

A system may comprise a localization unit for localizing the ablation electrode. The localization unit (**LU**) comprises a MEMS gyroscope which is attached to the basket catheter, the Microsoft Kinect System and a fluoroscopy system. An Inertia Monitoring Unit (**IMU**) for inclusion in the proposed system includes motion sensors and a MEMS gyroscope incorporated at each of the electrodes on each of the arms or splines of the catheter, which permits point by point localization of the catheter tip position and also an improved stability of the catheter system. The motion sensors may be any device capable of generating a signal that is indicative of the orientation or the rate of change of orientation of the sensor. The generated signal is preferably nearly proportional to the orientation or rate of change of the orientation of the sensor, but other dependencies are possible. For example, a sensor may be a liquid level pendulous tilt sensor, a physical gyroscope, a solid-state gyroscope, an accelerometer, or a pair of proximity sensors arranged in a line and separated by a known distance. Lightweight accelerometers and lightweight body sway sensors, such as velocity transducers or sensors may also be used or included as part of the sensors. In addition, micro-electro-mechanical systems (MEMS) accelerometers, piezoelectric accelerometers, or other rotation and/or linear accelerometers can be used. In various cases, a solid-state gyroscope is used with a liquid level tilt sensor. The liquid level tilt sensor may be used to correct for drift in the solid-state gyroscope.

One system includes a MEMS gyroscope because of its small size and low cost. A plurality of motion sensors and/or gyroscope(s) can monitor the position of the ablation catheter and feed signals to a processor. Preferably, the processor can perform a 3 x 3 matrix multiplication to computationally determine the roll, pitch and yaw. In one case, the motion processor then transmits the results from this computation to a holographic projection system, such as the Microsoft Kinect® system. The data from the computation will include x y and z coordinates which will be plotted into a holographic projection. In one case, at each point where x y and z coordinates are being generated, NIR, Raman, Fluorescence spectroscopy and OCT can be performed to collect depth data, which represent the depth of the muscle tissue at a particular point. The processor will input the data from the Raman spectroscopy and calculate the depth data and use the depth data to translate the x y and z coordinates into an outer shell of the cardiac silhouette, creating a 4D image of the heart. At the beginning of the ablation procedure, fluoroscopy images will be taken in the right anterior oblique and the left anterior oblique views. The LAO and RAO views will be used to reconstruct a 3D image. A computer algorithm will then transform the 3D data into Cartesian coordinates (x,y and z data). The basket catheter is then placed in the pulmonary vein/renal artery and a fluoroscopy image is taken. This will serve as the initialization point for the MEMS gyroscope. The location of the eight electrodes is therefore registered by the gyroscope. The gyroscope requires no external reference once it is initialized. As ablation is carried out, depth information from spatially offset diffuse reflectance spectroscopy is integrated with the 3D data to generate a 4D image.

For example, at the beginning of the procedure, excitation of the tissue by the illumination optic fiber is performed and scanning is performed by the receiving optic fiber by translating the receiving optic fiber with a motorized linear galvanometer. The optical spectra obtained in real time are compared to stored optical spectra for fat. The correlation between actual depth of the epicardial fat layer and the spatial offset is determined histologically in ex vivo and in vivo experiments. The depth of the tissue at which the epicardial fat layer is detected relates to the spatial offset between the illuminating and the receiving optic fibers and this information is relayed to the gyroscope which registers this as depth information on the 3D Cartesian coordinate system. The spatial offset is then adjusted to detect the muscle layer that is just superficial to the epicardial fat layer, again by comparing the optical spectra from the live on line data with stored optical spectra for muscle. The spatial offset at this depth is registered by the gyroscope. Ablation is then carried out.

The present methods are adapted to monitor the rate of change of reflectance as thermal denaturation is occurring. The rate of change of reflectance corresponds to the proportion of tissue that is being denatured. For a given temperature and a given rate constant, we can determine the time duration needed for 100% tissue denaturation to occur. Therefore, at each time point, the method is adapted to indicate the proportion of tissue that has been denatured. This information can be calculated to give the depth. This depth information is sent to the gyroscope, which then displays on the user interface the progression of the zone of damage as an M mode image. Once the first set of ablation is done, the location of the first set of 8 ablation points is registered by the gyroscope and the basket is rotated clockwise or counterclockwise to the ablation. Using the preferred configuration of 8 arms/splines, 2 or 3 rotations may be required to achieve a circumferential ablation around each pulmonary vein. Alternatively, in another case, the apparatus may be adapted to inclucle a basket with between 16 to 20 splines to achieve circumferential ablation with one-shot ablation.

The illustrated methods are only preferred examples. The invention is defined by the appended claims.

**Claims**

1. An ablation catheter apparatus (1000) comprising:

   an elongated body member having a proximal end, and a distal end;
   a basket (1070) at the distal end, the basket comprising a plurality of radially expanding splines (1020), the plurality of radially expanding splines (1020) including at least a first radially expanding spline (1020), a second radially expanding spline (1020), and a third radially expanding spline (1020); a first electrode (1050) located on the first radially expanding spline (1020), the first electrode (1050) configured to apply energy to tissue (2000, 2010, 2020, 2030, 3000) adjacent to the first radially expanding spline (1020) during an ablation procedure; and a second electrode (1050) located on the second radially expanding spline 1020, the second electrode 1050 configured to apply energy to tissue 2000, 2010, 2020, 2030, 3000 adjacent to the second radially expanding spline 1020 during the ablation procedure; whereby at least one of the first radially expanding spline (1020), the second radially expanding spline (1020), or the third radially expanding spline (1020) includes:

      at least one aperture (1040); a lumen;
      an optical emitting element (1025, 1027, 1028) inside the lumen configured to emit optical radiation through the aperture (1040) and into adjacent tissue during the ablation procedure; and

an optical receiving element (1025, 1027, 1028) inside the lumen configured to collect optical radiation through the aperture (1040) during the ablation procedure, the optical radiation indicating characteristics of adjacent tissue (2000, 2010, 2020, 2030, 3000) during the ablation procedure;

wherein the optical receiving element (1025, 1027, 1028) and the optical emitting element (1025, 1027, 1028) are arranged such that there is a spatial offset between the optical receiving element and the optical emitting element along a longitudinal axis of the lumen;

and wherein the optical receiving element (1025, 1027, 1028) is connected to a processing element, **characterized in that** the processing element is configured to process the optical radiation collected by the optical receiving element (1025, 1027, 1028) using spatially offset diffuse reflectance spectroscopy; and **in that** the processing element is configured to: automatically calculate the rate of thermal denaturation of the tissue adjacent to the plurality of radially expanding splines, and extract a rate constant for the rate of thermal denaturation when the tissue adjacent to plurality of radially expanding splines is sixty three percent denatured.

2. The apparatus of claim 1 wherein the optical receiving element (1025, 1027, 1028) and the optical emitting element (1025, 1027, 1028) are configured for movement relative to one another along the longitudinal axis of the lumen such that the spatial offset between the optical receiving element and the optical emitting element is adjustable.

3. The apparatus of claim 2 wherein at least one of the optical receiving element (1025, 1027, 1028) or the optical emitting element (1025, 1027, 1028) is coupled to an actuating device, the actuating device being configured to cause the relative movement between the optical receiving element and the optical emitting element.

4. The apparatus of any preceding claim, wherein the processing element is configured to generate optical spectra of the optical radiation collected by the optical receiving element (1025, 1027, 1028) and compare the generated optical spectra of the optical radiation collected by the optical receiving element to reference optical spectra for at least one of following tissue types: fat, nerve, muscle, or collagen.

5. The apparatus of any preceding claim, wherein the processing element is configured to determine that adjacent tissue is at least one of the following tissue types: fat, nerve, muscle, or collagen based at least on the comparison between the generated optical spectra and the reference optical spectra.

6. The apparatus of any preceding claim, wherein the processing element is configured to compare the generated optical spectra of the optical radiation collected by the optical receiving element to reference optical spectra for tissue fluid.

7. The apparatus of claim 6, wherein the processing element is configured to determine that adjacent tissue contains tissue fluid based at least on the comparison between the generated optical spectra and the reference optical spectra.

8. The apparatus of claim 5, 6 or 7, wherein the optical receiving element (1025, 1027, 1028) and the optical emitting element (1025, 1027, 1028) are configured for movement relative to one another along the longitudinal axis of the lumen such that the spatial offset between the optical receiving element and the optical emitting element is adjustable.

9. The apparatus of any preceding claim, wherein the spatial offset between the optical receiving element (1025, 1027, 1028) and the optical emitting element (1025, 1027, 1028) has a magnitude of at least 10 mm.

10. The apparatus of any preceding claim, wherein the optical emitting element (1025, 1027, 1028) and the optical receiving element (1025, 1027, 1028) have a spatial offset of at least 10 mm and are configured such that the optical receiving element (1025, 1027, 1028) collect near infrared signatures from muscle tissue, the near-infrared signatures to represent the extent of fibrosis within the muscle tissue.

11. The apparatus of any preceding claim, wherein the optical receiving element (1025, 1027, 1028) is connected to a processing element configured to process the optical radiation collected by the optical receiving element (1025, 1027, 1028) using at least one of the following techniques: near-infrared spectroscopy, Raman spectroscopy, reflectance spectroscopy, optical coherence tomography, or a technique based on birefringence.

12. The apparatus of any preceding claim, wherein the aperture (1040) is located on the first electrode (1050) or the second electrode (1050).

13. The apparatus of any preceding claim, wherein the ablation catheter (1000) is connected to an energy source configured to provide energy to the first and second electrodes (1050) such that the first electrode (1050) can apply energy to tissue adjacent to the first radially expanding spline (1020) and the second electrode (1050) can apply energy to tissue adjacent to the second radially expanding spline (1020) substantially simultaneously.

14. The apparatus of any preceding claim, wherein the ablation catheter (1000) is connected to an optical radiation source configured to provide optical radiation to a first, second, and third optical illuminating elements (1025, 1027, 1028) such that the first, second, and third optically illuminating elements (1025, 1027, 1028) substantially simultaneously emit optical radiation.

**Patentansprüche**

1. Ablationskathetervorrichtung (1000), die Folgendes umfasst:

ein langgestrecktes Körperelement mit einem proximalen Ende und einem distalen Ende; einen Korb (1070) an dem distalen Ende, wobei der Korb eine Vielzahl von sich radial spreizenden Stäben (1020) umfasst, wobei die Vielzahl von sich radial spreizenden Stäben (1020) mindestens einen ersten sich radial spreizenden Stab (1020), einen zweiten sich radial spreizenden Stab (1020) und einen dritten sich radial spreizenden Stab (1020) umfasst; eine erste Elektrode (1050), die sich an dem ersten sich radial spreizenden Stab (1020) befindet, wobei die erste Elektrode (1050) dazu konfiguriert ist, während eines Ablationsvorgangs Energie an dem ersten sich radial spreizenden Stab (1020) benachbartes Gewebe (2000, 2010, 2020, 2030, 3000) anzulegen; und eine zweite Elektrode (1050), die sich an dem zweiten sich radial spreizenden Stab (1020) befindet, wobei die zweite Elektrode (1050) dazu konfiguriert ist, während des Ablationsvorgangs Energie an dem zweiten sich radial spreizenden Stab (1020) benachbartes Gewebe (2000, 2010, 2020, 2030, 3000) anzulegen; wodurch mindestens einer von dem ersten sich radial spreizenden Stab (1020), dem zweiten sich radial spreizenden Stab (1020) und dem dritten sich radial spreizenden Stab (1020) Folgendes umfasst:

mindestens ein Loch (1040); ein Lumen; ein optisches Emitterelement (1025, 1027, 1028) in dem Lumen, das dazu konfiguriert ist, während des Ablationsvorgangs optische Strahlung durch das Loch (1040) und in benachbartes Gewebe auszustrahlen; und

ein optisches Empfangselement (1025, 1027, 1028) in dem Lumen, das dazu konfiguriert ist, während des Ablationsvorgangs optische Strahlung durch das Loch (1040) zu erfassen, wobei die optische Strahlung während des Ablationsvorgangs Eigenschaften benachbarten Gewebes (2000, 2010, 2020, 2030, 3000) angibt; wobei das optische Empfangselement (1025, 1027, 1028) und das optische Emitterelement (1025, 1027, 1028) derart angeordnet sind, dass ein räumlicher Versatz entlang einer Längsachse des Lumens zwischen dem optischen Empfangselement und dem optischen Emitterelement vorliegt; und wobei das optische Empfangselement (1025, 1027, 1028) mit einem Verarbeitungselement verbunden ist, **dadurch gekennzeichnet, dass** das Verarbeitungselement dazu konfiguriert ist, die von dem optischen Empfangselement (1025, 1027, 1028) erfasste optische Strahlung unter Verwendung von räumlich versetzter diffuser Reflexionsspektroskopie zu verarbeiten; und dadurch, dass das Verarbeitungselement dazu konfiguriert ist: die Geschwindigkeit der thermischen Denaturierung des der Vielzahl von sich radial spreizenden Stäben benachbarten Gewebes automatisch zu berechnen und eine Geschwindigkeitskonstante für die Geschwindigkeit der thermischen Denaturierung auszuziehen, wenn das der Vielzahl von sich radial spreizenden Stäben benachbarte Gewebe zu dreiundsechzig Prozent denaturiert ist.

2. Vorrichtung nach Anspruch 1, wobei das optische Empfangselement (1025, 1027, 1028) und das optische Emitterelement (1025, 1027, 1028) zur Bewegung relativ zueinander entlang der Längsachse des Lumens konfiguriert sind, sodass der räumliche Versatz zwischen dem optischen Empfangselement und dem optischen Emitterelement verstellbar ist.

3. Vorrichtung nach Anspruch 2, wobei das optische Empfangselement (1025, 1027, 1028) und/oder das optische Emitterelement (1025, 1027, 1028) an eine Betätigungseinrichtung gekoppelt ist, wobei die Betätigungseinrichtung dazu konfiguriert ist, die Relativbewegung zwischen dem optischen Empfangselement und dem optischen Emitterelement zu bewirken.

4. Vorrichtung nach einem der vorangehenden An-

sprüche, wobei das Verarbeitselement dazu konfiguriert ist, optische Spektren der von dem optischen Empfangselement (1025, 1027, 1028) erfassten optischen Strahlung zu erzeugen, und die erzeugten optischen Spektren der von dem optischen Empfangselement erfassten optischen Strahlung mit optischen Bezugsspektren für mindestens einen der folgenden Gewebetypen zu vergleichen: Fett, Nerv, Muskel oder Collagen.

5. Vorrichtung nach einem der vorangehenden Ansprüche, wobei das Verarbeitselement dazu konfiguriert ist, basierend mindestens auf dem Vergleich zwischen den erzeugten optischen Spektren und den optischen Bezugsspektren zu bestimmen, dass es sich bei benachbartem Gewebe um mindestens einen der folgenden Gewebetypen handelt: Fett, Nerv, Muskel oder Collagen.

6. Vorrichtung nach einem der vorangehenden Ansprüche, wobei das Verarbeitselement dazu konfiguriert ist, die erzeugten optischen Spektren der von dem optischen Empfangselement erfassten optischen Strahlung mit optischen Bezugsspektren für Gewebeflüssigkeit zu vergleichen.

7. Vorrichtung nach Anspruch 6, wobei das Verarbeitungselement dazu konfiguriert ist, basierend mindestens auf dem Vergleich zwischen den erzeugten optischen Spektren und dem optischen Bezugsspektren zu bestimmen, dass benachbartes Gewebe Gewebeflüssigkeit enthält.

8. Vorrichtung nach Anspruch 5, 6 oder 7, wobei das optische Empfangselement (1025, 1027, 1028) und das optische Emitterelement (1025, 1027, 1028) zur Bewegung relativ zueinander entlang der Längsachse des Lumens konfiguriert sind, sodass der räumliche Versatz zwischen dem optischen Empfangselement und dem optischen Emitterelement verstellbar ist.

9. Vorrichtung nach einem der vorangehenden Ansprüche, wobei der räumliche Versatz zwischen dem optischen Empfangselement (1025, 1027, 1028) und dem optischen Emitterelement (1025, 1027, 1028) eine Größe von mindestens 10 mm aufweist.

10. Vorrichtung nach einem der vorangehenden Ansprüche, wobei das optische Emitterelement (1025, 1027, 1028) und das optische Empfangselement (1025, 1027, 1028) einen räumlichen Versatz von mindestens 10 mm aufweisen und derart konfiguriert sind, dass das optische Empfangselement (1025, 1027, 1028) Nahinfrarotsignaturen von Muskelgewebe erfasst, wobei die Nahinfrarotsignaturen das Ausmaß von Fibrose in dem Muskelgewebe repräsentieren.

11. Vorrichtung nach einem der vorangehenden Ansprüche, wobei das optische Empfangselement (1025, 1027, 1028) mit einem Verarbeitselement verbunden ist, das dazu konfiguriert ist, die von dem optischen Empfangselement (1025, 1027, 1028) erfasste Strahlung unter Verwendung mindestens einer der folgenden Techniken zu verarbeiten: Nahinfrarotspektroskopie, Ramanspektroskopie, Reflexionsspektroskopie, optischer Kohärenztomographie oder einer auf Doppelbrechung basierenden Technik.

12. Vorrichtung nach einem der vorangehenden Ansprüche, wobei sich das Loch (1040) an der ersten Elektrode (1050) oder der zweiten Elektrode (1050) befindet.

13. Vorrichtung nach einem der vorangehenden Ansprüche, wobei der Ablationskatheter (1000) mit einer Energiequelle verbunden ist, die dazu konfiguriert ist, der ersten und der zweiten Elektrode (1050) Energie bereitzustellen, sodass die erste Elektrode (1050) Energie an dem ersten sich radial spreizenden Stab (1020) benachbartes Gewebe anlegen kann und die zweite Elektrode (1050) im Wesentlichen gleichzeitig Energie an dem zweiten sich radial spreizenden Stab (1020) benachbartes Gewebe anlegen kann.

14. Vorrichtung nach einem der vorangehenden Ansprüche, wobei der Ablationskatheter (1000) mit einer Quelle optischer Strahlung verbunden ist, die dazu konfiguriert ist, einem ersten, einem zweien und einem dritten optischen Beleuchtungselement (1025, 1027, 1028) optische Strahlung bereitzustellen, sodass das erste, das zweite und das dritte optische Beleuchtungselement (1025, 1027, 1028) im Wesentlichen gleichzeitig optische Strahlung ausstrahlen können.

## Revendications

1. Appareil formant cathéter d'ablation (1000) comportant :

   un élément formant corps allongé ayant une extrémité proximale, et une extrémité distale ;
   un panier (1070) au niveau de l'extrémité distale, le panier comportant une pluralité de cannelures expansibles dans le sens radial (1020), la pluralité de cannelures expansibles dans le sens radial (1020) comprenant au moins une première cannelure expansible dans le sens radial (1020), une deuxième cannelure expansible dans le sens radial (1020), et une troisième cannelure expansible dans le sens radial (1020) ;
   une première électrode (1050) située sur la première cannelure expansible dans le sens radial (1020), la première électrode (1050) étant configurée pour appliquer de l'énergie sur le tissu (2000, 2010, 2020, 2030, 3000) adjacent par rapport à la première cannelure expansible dans le sens radial (1020) au cours d'une procédure d'ablation ; et

une deuxième électrode (1050) située sur la deuxième cannelure expansible dans le sens radial (1020), la deuxième électrode (1050) étant configurée pour appliquer de l'énergie sur le tissu (2000, 2010, 2020, 2030, 3000) adjacent par rapport à la deuxième cannelure expansible dans le sens radial (1020) au cours de la procédure d'ablation ;

ce par quoi au moins l'une parmi la première cannelure expansible dans le sens radial (1020), la deuxième cannelure expansible dans le sens radial (1020), ou la troisième cannelure expansible dans le sens radial (1020) comprend :

au moins une ouverture (1040) ;
une lumière ;
un élément d'émission optique (1025, 1027, 1028) à l'intérieur de la lumière, configuré pour émettre un rayonnement optique au travers de l'ouverture (1040) et jusque dans le tissu adjacent au cours de la procédure d'ablation ; et
un élément de réception optique (1025, 1027, 1028) à l'intérieur de la lumière, configuré pour collecter un rayonnement optique au travers de l'ouverture (1040) au cours de la procédure d'ablation, le rayonnement optique indiquant des caractéristiques du tissu adjacent (2000, 2010, 2020, 2030, 3000) au cours de la procédure d'ablation ;
dans lequel l'élément de réception optique (1025, 1027, 1028) et l'élément d'émission optique (1025, 1027, 1028) sont agencés de telle sorte qu'il y a un décalage spatial entre l'élément de réception optique et l'élément d'émission optique le long d'un axe longitudinal de la lumière ;
et dans lequel l'élément de réception optique (1025, 1027, 1028) est connecté à un élément de traitement, **caractérisé en ce que** l'élément de traitement est configuré pour traiter le rayonnement optique collecté par l'élément de réception optique (1025, 1027, 1028) en utilisant la spectroscopie par réflectance diffuse à décalage spatial ; et
**en ce que** l'élément de traitement est configuré pour : calculer automatiquement la vitesse de dénaturation thermique du tissu adjacent par rapport à la pluralité de cannelures expansibles dans le sens radial, et extraire une constante de vitesse pour la vitesse de dénaturation thermique quand le tissu adjacent par rapport à la pluralité de cannelures expansibles dans le sens radial est dénaturé à soixante trois pour cent.

**2.** Appareil selon la revendication 1, dans lequel l'élément de réception optique (1025, 1027, 1028) et l'élément d'émission optique (1025, 1027, 1028) sont configurés à des fins de mouvement relatif l'un par rapport à l'autre le long de l'axe longitudinal de la lumière de telle sorte que le décalage spatial entre l'élément de réception optique et l'élément d'émission optique est ajustable.

**3.** Appareil selon la revendication 2, dans lequel au moins l'un parmi l'élément de réception optique (1025, 1027, 1028) ou l'élément d'émission optique (1025, 1027, 1028) est accouplé à un dispositif d'actionnement, le dispositif d'actionnement étant configuré pour causer le mouvement relatif entre l'élément de réception optique et l'élément d'émission optique.

**4.** Appareil selon l'une quelconque des revendications précédentes, dans lequel l'élément de traitement est configuré pour générer des spectres optiques du rayonnement optique collecté par l'élément de réception optique (1025, 1027, 1028) et pour comparer les spectres optiques générés du rayonnement optique collecté par l'élément de réception optique par rapport à des spectres optiques de référence pour au moins l'un des types de tissus suivants : le tissu adipeux, le tissu nerveux, le tissu musculaire, ou le collagène.

**5.** Appareil selon l'une quelconque des revendications précédentes, dans lequel l'élément de traitement est configuré pour déterminer que le tissu adjacent est au moins l'un des types de tissus suivants : le tissu adipeux, le tissu nerveux, le tissu musculaire, ou le collagène en se basant sur au moins la comparaison entre les spectres optique générés et les spectres optiques de référence.

**6.** Appareil selon l'une quelconque des revendications précédentes, dans lequel l'élément de traitement est configuré pour comparer les spectres optiques générés du rayonnement optique collecté par l'élément de réception optique par rapport aux spectres optiques de référence en matière de liquide tissulaire.

**7.** Appareil selon la revendication 6, dans lequel l'élément de traitement est configuré pour déterminer que le tissu adjacent contient du liquide tissulaire en se basant au moins sur la comparaison entre les spectres optiques générés et les spectres optiques de référence.

**8.** Appareil selon la revendication 5, la revendication 6 ou la revendication 7, dans lequel l'élément de réception optique (1025, 1027, 1028) et l'élément d'émission optique (1025, 1027, 1028) sont configurés à des fins de mouvement relatif l'un par rapport à l'autre le long de l'axe longitudinal de la lumière de telle sorte que le décalage spatial entre l'élément de réception optique et l'élément d'émission optique est ajustable.

**9.** Appareil selon l'une quelconque des revendications précédentes, dans lequel le décalage spatial entre l'élément de réception optique (1025, 1027, 1028) et l'élément d'émission optique (1025, 1027, 1028) a une ampleur d'au moins 10 mm.

**10.** Appareil selon l'une quelconque des revendications précédentes, dans lequel l'élément d'émission optique (1025, 1027, 1028) et l'élément de réception optique (1025, 1027, 1028) ont un décalage spatial d'au moins 10 mm et sont configurés de telle sorte que l'élément de réception optique (1025, 1027, 1028) collecte des signatures dans le proche infrarouge en provenance du tissu musculaire, les signatures dans le proche infrarouge représentant l'étendue de la fibrose dans le tissu musculaire.

**11.** Appareil selon l'une quelconque des revendications précédentes, dans lequel l'élément de réception optique (1025, 1027, 1028) est connecté à un élément de traitement configuré pour traiter le rayonnement optique collecté par l'élément de réception optique (1025, 1027, 1028) en utilisant au moins l'une des techniques suivantes : la spectroscopie dans le proche infrarouge, la spectroscopie de Raman, la spectroscopie par réflectance, la tomographie par cohérence optique, ou une technique basée sur la biréfringence.

**12.** Appareil selon l'une quelconque des revendications précédentes, dans lequel l'ouverture (1040) est située sur la première électrode (1050) ou la deuxième électrode (1050).

**13.** Appareil selon l'une quelconque des revendications précédentes, dans lequel le cathéter d'ablation (1000) est connecté à une source d'énergie configurée pour fournir de l'énergie aux première et deuxième électrodes (1050) de telle sorte que la première électrode (1050) peut appliquer de l'énergie sur le tissu adjacent par rapport à la première cannelure expansible dans le sens radial (1020) et la deuxième électrode (1050) peut appliquer de l'énergie sur le tissu adjacent par rapport à la deuxième cannelure expansible dans le sens radial (1020) de manière sensiblement simultanée.

**14.** Appareil selon l'une quelconque des revendications précédentes, dans lequel le cathéter d'ablation (1000) est connecté à une source de rayonnement optique configurée pour fournir un rayonnement optique à des premier, deuxième, et troisième éléments d'éclairage optiques (1025, 1027, 1028) de telle sorte que les premier, deuxième, et troisième éléments d'éclairage optiques (1025, 1027, 1028) peuvent émettre un rayonnement optique de manière sensiblement simultanée.

## FIG.1

$M_{xy} = 1 - \exp(-t/400)$

63%

400 ms

FIG.1a

FIG.1b

FIG.2

FIG.3

*1000*

*FIG.4*

FIG.5

1020

1020

FIG.6

FIG.7

1080    1050

1040

1051    FIG.8    1020

1040    1020    1080

1051    FIG.9    1050

FIG.10

1027

1020

1028

FIG.11

1051

*1025*

*1020*

0.405 mm

1 mm

Optic fibers
within
PVC/polyamide
sleeve

*1025*

*FIG.12*

1029

1024

1040

EFL

1027

1020

3000

1050

1029

1028

FIG.13

FIG.14

Reference
signal

Light Source

Illumination Optic Fibers

Detection optical fibers

Photomultiplier

Detection optical fiber
displacement actuator

Filter

Optical fiber
displacement
control signals

Signal

Lock-in amplifier

Computer

FIG.15

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2010063492 A1
- WO 2012049621 A1, Harks

**Non-patent literature cited in the description**

- *Heart Rhythm,* January 2012, vol. 9 (1), 18-23
- **CUI, W. ; KUMAR, C. ; CHANCE, B.** Experimental study of migration depth for the photons measured at sample surface. I. Time resolved spectroscopy and imaging. *Proc. SPIE Int. Soc. Opt. Eng.,* 1991, vol. 1431, 180-191
- Clinical optical dose measurement for PDT : Invasive and non-invasive techniques. **R BAYS et al.** Future Trends in Biomedical Applications of Lasers. SPIE, 1991, vol. 1525, 397-408
- **DAVID M HAALAND.** Partial Least-Squares Methods for Spectral Analyses. 1. Relation to Other Quantitative Calibration Methods and the Extraction of Qualitative Information. *Analytical Chemistry,* June 1988, vol. 60 (11), 1193-1202